# EUROPEAN PATENT SPECIFICATION

(11) **EP 1 539 803 B1**
(45) Date of publication and mention of the grant of the patent: **28.02.2007**
(21) Application number: 03771244.5
(22) Date of filing: 18.07.2003
(51) Int. Cl.: C07K 7/54, C07K 7/64, A61K 38/12, G01N 33/68

(54) **FLUORO-ALKYL-CYCLOPEPTIDE DERIVATIVES HAVING ANTI-INTEGRIN ACTIVITY**
FLUORALKYLCYCLOPEPTIDDERIVATE MIT ANTIINTEGRINWIRKUNG
DERIVES DE FLUORO-ALKYL-CYCLOPEPTIDE DOTES D'UNE ACTIVITE ANTI-INTEGRINE

(30) Priority: 29.07.2002 IT RM20020402
(43) Date of publication of application: 15.06.2005
(73) Proprietor: SIGMA-TAU Industrie Farmaceutiche Riunite S.p.A., 00144 Roma (IT)
(72) Inventor: DAL POZZO, Alma, Istituto Scientifico "G. Ronzoni", I-20133 Milano (IT); GIANNINI, G., Sigma-Tau Ind. Farm. Riunite S.P.A., I-00040 Pomezia (IT); PISANO, C., Sigma-Tau Ind. Farm. Riunite S.P.A., I-00040 Pomezia (IT)
(74) Representative: Spadaro, Marco
(86) International application number: PCT/IT2003/000446
(87) International publication number: WO 2004/011487

(56) References cited:
- EP-A- 0 632 053
- DE-A- 19 613 933
- DE-A- 19 725 368
- US-A- 5 773 412
- DAL POZZO A ET AL: "Synthesis of RGD Analogues Containing alpha-Tfm-Arginine as Potential Fibrinogen Receptor Antagonists" TETRAHEDRON, ELSEVIER SCIENCE PUBLISHERS, AMSTERDAM, NL, vol. 54, no. 22, 28 May 1998 (1998-05-28), pages 6019-6028, XP004118448 ISSN: 0040-4020 cited in the application
- HAUBNER R ET AL: "STRUCTURAL AND FUNCTIONAL ASPECTS OF RGD-CONTAINING CYCLIC PENTAPEPTIDES AS HIGHLY POTENT AND SELECTIVE INTEGRIN ALPHAVBETA3 ANTAGONISTS" JOURNAL OF THE AMERICAN CHEMICAL SOCIETY, AMERICAN CHEMICAL SOCIETY, WASHINGTON, DC, US, vol. 118, no. 32, 14 August 1996 (1996-08-14), pages 7461-7472, XP002026923 ISSN: 0002-7863

## Description

The invention described herein relates to fluoro-alkyl-cyclopeptide derivatives endowed with anti-integrin activity, particularly cyclic peptide compounds containing fluoro-alkyl groups on the nitrogen of the peptide bond and/or in position C-α, as indicated in formula (I) here below. The invention described herein also relates to processes for the preparation of said compounds, their use as medicaments, particularly as inhibitors of the integrin receptors, useful as antiangiogenic and antimetastatic agents and to pharmaceutical compositions containing them.

### Background to the invention

The integrins are a class of receptors involved in the cell adhesion phenomenon. They are glycoproteins consisting of two subunits α and β, combined to give different families. For greater details, see *Kessler, H., et al., Angew. Chem. Int. Ed. Engl., 1997, 36, 1374-89.*

All the integrins have a "universal cell recognition site" capable of recognising the common peptide sequence Arg-Gly-Asp, also known as RGD, from the one-letter symbols that identify the three amino acids, though every integrin preferentially recognises a different conformation of this tripeptide (*Kessler, H., et al., J. Am. Chem. Soc., **1996,** 118, 7461-72).*

The family of the β₁ integrins plays an important role in the organisation of the tissues and the β₂ integrins are important for the immune system, while the β₃ integrins regulate the coagulation process and angiogenesis.

One goal of pharmaceutical chemistry is to make available to the physician compounds capable of interacting with the integrin family, but selectively on the various subtypes, in view of the diversity of roles that each of them plays at the physiopathological level.

The invention described herein is aimed at the integrins involved in angiogenesis mechanisms.

The action of different growth factors stimulates the expression of integrin αᵥβ₃ (vitronectin receptor) on endothelial cells. During the consequent migration of the endothelial cells in the direction of angiogenesis stimulation, the membrane with the αᵥβ₃ integrin receptor binds the tripeptide sequence RGD present in the various forms on the extracellular matrix. This binding leads to an accumulation of proteins - of the thalin, paxilin, α-actinin, tensin, and vinculin types - of the cytoskeleton. This favours the process of migration, acting as an endothelial cell survival signal, with the formation of new blood vessels. The administration of soluble RGD analogues impedes the build-up of proteins on the receptors and leads to programmed cell death (apoptosis), counteracting the migration of endothelial cells and preventing neovascularisation *(Giannis, A., et al., Angew. Chem. Int. Ed. Engl., 1997, 36, 588-90*)*.*

Among the many molecules selectively involved in angiogenesis, the integrins constitute a promising target in cancer therapy and in all those diseases responsible for uncontrolled neovascularisation.

An initial scientific study of the subject *(Saiki, I., et al., Jpn. J. Cancer Res., 1990, 81:668-675)* reports the action of peptides containing an RGD sequence recognising integrin, thus inhibiting angiogenesis in tumours.

The RGD tripeptide is present in natural ligands of these receptors, such as vitronectin, fibronectin and fibrinogen.

More recent studies have shown that type αᵥβ₃ and αᵥβ₅ integrins increase in the angiogenesis of endothelial cell tumours and that inhibition of αᵥ integrins by means of antibodies, RGD cyclic peptides and RGD peptidomimetic agents, can block neovascularisation (*Arap, W., et al., Current Opinion in Oncology, 1998, 10:560-565*). β₁ integrins (α₁β₁ and α₂β₁), too, may also play a role in angiogenesis, though their role has yet to be thoroughly studied.

The systemic administration of an anti-αᵥβ₃ antibody, e.g. the LM609 antibody (Vitaxin), blocks tumour angiogenesis and reduces the growth and invasive properties of human carcinoma of the breast *(Brooks, P.C., et al., J. Clin. Invest., 1995, 96:1815-22*).

Many integrins can be inhibited by small peptides incorporating the RGD sequence. The incorporation of this sequence in penta- or hexapeptide cycles containing D-amino acids usually leads to molecules that are potent, selective integrin inhibitors *(Haubnev, R., et al., J. Am. Chem. Soc., 1996, 118:7881-91).*

Vitronectin is a protein of the vascular matrix and a selective antagonist of the αᵥβ₃ receptors, while fibrinogen, another protein, presents selective binding to the α_{IIb}β₃ receptors.

To date, the search for RGD analogues has been directed mainly towards antagonists of the α_{IIb}β₃ receptors that are potent and selective and can be administered orally. Some of these non-peptide RGD analogues, used as anticoagulants, are currently being investigated in clinical trials.

As antiangiogenic agents, on the other hand, what are needed are RGD analogues capable of selectively inhibiting the αᵥβ₃ and/or αᵥβ₅ receptors without affecting the α_{IIb}β₃ receptors.

As regards examples of compounds inhibiting the αᵥβ₃ receptors and their applications, see EP 1 077 218, filed in the name of the applicant, to which specific reference is made, also for further discussion of the state of the art.

Examples of cyclic peptide structures containing the Arg-Gly-Asp (RGD) sequence are described in EP 0 596 350, Merck Patent; *Wermuth, J., et al., J. Am. Chem. Soc., 1997, 119(6), 1328-1335;* US 5.705.481, Merck Patent; WO 99/58162, Du Pont Pharmaceuticals; *Liu, S., et al., Bioconjugate Chemistry (2001), 12(4), 559-568;* WO 01/097860.

DE 197 25 368 discloses cyclic peptides containing the RGD sequence. These peptides may contain a Phe, optionally fluorinated on the phenyl ring. No special technical meaning is attributed to fluorine atom and the compounds interact with the integrin receptors with no particular selectivity.

One of the aims of the invention described herein is to provide selective agonists for the αᵥβ₃ receptors that can be administered orally, which is a useful characteristic for long-term therapies.

Examples of natural compounds containing a fluorine atom are rare. This element, thanks to its physicochemical properties (dimensions, electronegativity, etc.), may endow biologically active organic compounds with particular characteristics.

In recent years, with the availability of more manageable fluorinating reagents, organic derivatives with promising characteristics have been prepared, e.g.: amino acid substrates of pyridoxal-dependent enzymes (transaminases and decarboxylases) act as specific inactivators when they are fluorinated; fluorinated pyrimidines exert anticancer activity; trifluoromethylated analogues of captopril have less than nM activity; fluorinated analogues of natural anthracyclines are much more active as anticancer agents (*Giannini, G., Current Medicinal Chem., 2002,* 9:1867-93); trifluoromethylated enkephalin analogues have increased their potency more than 10,000 fold. Fluorinated linear analogues of RGD are described in *A. Dal Pozzo, et al., J. Chem. Res.* (S) 468-469 (1999). See also *Ojima, I., Organofluorine Compounds in Medicinal Chemistry and Biomedical Applications; R. Filler (ed), 1993 - Elsevier Science Publisher; Ojima, I. et al., Biomedical Frontiers of Fluorine Chemistry - ACS Symposium Series 639 (1996); Sewald, N. et al., Amino Acids (1995) 8:187-194.*

The alkylation of an amino acid is a very important aspect of pharmaceutical chemistry. Also in the field of intergrin inhibitors there are examples of N-alkylation of amino acids (*Kessler, H., et al., J. Med. Chem., 1999, 42, 3033-40),* which have led to the product called EMD 121874 (Cilengitide), developed by Merck and currently undergoing phase II clinical trials.

Much less frequent is the α-alkylation approach, though it, too, is still potentially promising. It is known, in fact, that the incorporation of C^{α}-disubstituted amino acids in key positions of peptides may modify and stabilise the secondary structure (*Marshall, G.R., Int. J. Pept. Protein Res., 1998, 32, 544-5*). Moreover, the substitution of fluorine for hydrogen may affect the proteolytic stability and solubility of the peptide containing it (*Koksch, B., J. Pept. Sci., 1997, 3, 15*7-67).

The subsequent stage of alkylation is the fluoroalkylation of amino acids. In this case, too, there are examples of fluoroalkylation of linear oligopeptides *(Dal Pozzo, A., et al. Tetrahedron, 1998, 54: 6019-28; Koksch, B. et al. Biomedical Frontiers of Fluorine Chemistry; ACS Symposium Series 639, Chapter 3, 1996, 42-58).* Fluoralkylated peptides present the following advantages: protection of physiological peptidases, similar to alkyl; a substantial increase in the hydrophobic character (superior to most of the alkyl groups), which increases the bioavailability (absorption and distribution); stiffening of the conformation, due to the bulk (which in solution is greater than that of a simple methyl group), the unique ability to give hydrogen bonds, which, amongst other things, may cause restricted rotation around the carbamide bond.

Another aim of the present invention is to provide a process of synthesis of peptides containing, in a key position, fluoroalkylated amino acids in the C-α and/or N- position, and, despite the steric bulk and the electronic effects of the new structures produced, the discovery of the biological properties of these compounds, such as potent selective inhibitors of αᵥβ₃ and/or αᵥβ₅ integrin receptors.

### Summary of the invention

It has now been found that compounds of formula (I)

cyclo[NX₁-R₁-CO-NX₂-R₂-CO-NX₃-R₃-CO-NX₄-R₄-CO-NX₅-R₅-CO]

where:
R₁ is selected from:

   CH(CH₂)₃NHC(NH)NH₂; C[CHₙFₘ](CH₂)₃NHC(NH)NH₂
R₂ is the group CH₂; CH₂-CH₂; 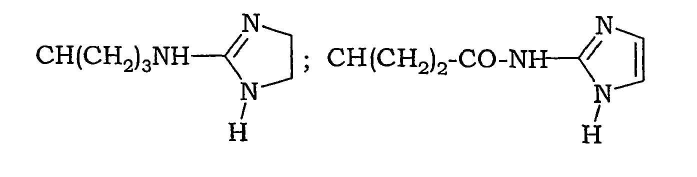
R₃ is selected from CHCH₂COOH; C[CHₙFₘ]CH₂-COOH;
R₄ is selected from CH-CH₂-Ph; C[CHₙFₘ]CH₂-Ph; CH-CH₂-(4-OH)Ph; CH-CH₂-(4-OMe)Ph; CH-CH₂-(4-F)Ph; CH-CH(OH)-Ph; C(CH₃)₂; CH-C(CH₃)₃; CH-CH₂-COOH; 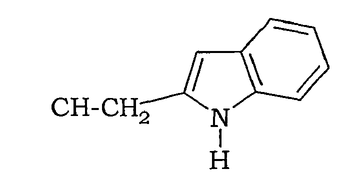
R₅ is selected from CH-CH₂-Ph; C[CHₙFₘ]CH₂-Ph; CH-CH(CH₃)₂; C[CHₙFₘ]CH(CH₃)₂; CH-C(CH₃)₃;
or, the NX₄-R₄-CO-NX₅-R₅-CO group is 3-aminomethyl-benzoyl n+m=3
X₁-X₅, which may be the same or different, are H, (CH₂)ₙ-CH₃; (CH₂)ₙ- 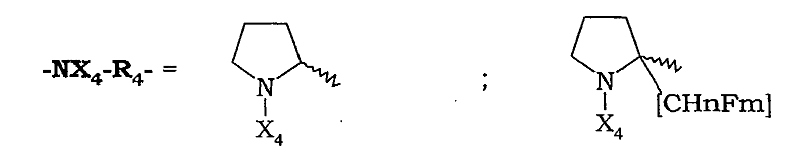 CHF₂; (CH₂)ₙ-CH₂F, (CH₂)ₙ-CF₃ where n= 0-3;
with the proviso that at least one α-fluoroalkylated amino acid is present in the formula (I) compound.
where each NX-R-CO amino acid can have an absolute type R or type S configuration; their individual enantiomers, diastereoisomers, the related mixtures, the pharmaceutically acceptable salts are selective inhibitors of the αᵥβ₃ and/or αᵥβ₅ integrin receptors.

Therefore, objects of the present invention are compounds of formula (I), as described above, a process for their preparation, their use as medicaments and pharmaceutical compositions containing them.

These and other objects of the present invention will be illustrated in detail, also by means of examples.

### Detailed description of the invention

According to the present invention, pharmaceutically acceptable salts are all those salts that the expert in the field is capable of preparing, without the acid or base used giving rise to unwanted effects, when said salts are used as medicaments.

In the context of the present invention, the following compounds are preferred:
c(Arg-Gly-Asp-D-Phe-(*R or* S)-Tfm-Phe) (ST1930/ST1931);
c(Arg-Gly-Asp-D-Phe-(*R*,S)- Dfm - Phe) (ST1932);
c(Arg-Gly-(*R* or S)-Tfm-Asp-D-Phe-Val) (ST2189/2190);
c(Arg-Gly-Asp-(*R* or S)-Tfm-Phe-Val) (ST2191/2192);
c(Arg-Gly-Asp-D-Phe-(*R* or S)-Tfm-Val) (ST2409/ST2410);
c(Arg-Gly-Asp-D-Phe-(*R* or *S*)-N-Me-Tfm-Phe.

In its broader aspect, the present invention provides a method for selectively inhibiting cellular adhesion mediated by αᵥβ₃ and αᵥβ₅ integrins to a ligand containing the RGD sequence. In another of its aspects, the purpose of the present invention is the use of a formula (I) compound for the preparation of a medicament useful for treating subjects affected by abnormal angiogenesis. Examples of use of the medicament according to the invention are the reduction of metastases and the treatment of retinopathy, acute kidney failure and osteoporosis.

In another of its aspects, the object of the present invention consists in the use of the abovementioned compounds as diagnostic agents. In particular, the compounds according to the present invention, when appropriately labelled, are useful for detecting and locating small tumour masses. Similarly, said labelled compounds are also useful for the analysis of arterial occlusion events such as strokes or myocardial infarcts.

Therefore, a further object of the present invention is the use of compounds of formula (I), as described above, for the preparation of diagnostic agents, particularly for the detection and location of tumours, and preferably small tumour masses, or of arterial occlusion events such as strokes or myocardial infarcts. Covered by the present invention are also diagnostic agents containing at least one formula (I) compound. As regards the labelling of the compounds according to the present invention, this is part of the normal expertise of the average technician in the field, who, on the basis of his or her specific knowledge, is capable of choosing the appropriate labelling agent and of derivatising the compound according to the invention. An example of an application for the compounds according to the present invention can be seen in WO 99/11590 and in the following references: *Su, Z.F., et al., Bioconjug. Chem. 2002 May-Jun; 13(3):561-70; Haubner, R., et al., Cancer Res. 2001 Mar 1;61(5):1781-5; Haubner R, et al., J. Nucl. Med. 2001 Feb; 42(2):326-36; van Hagen, P.M, et al., Int. J. Cancer 2000 Aug 20; 90(4):186-98; Sivolapenko, G.B., et al., Eur. J. Nucl. Med. 1998 Oct; 25(10):1383-9; Pearson, D.A., et al., J. Med. Chem. 1996 Mar 29; 39(7):1372-82.*

From the chemical point of view, the present invention consists in obtaining cyclised derivatives of a peptide structure containing a number of non-natural amino acids (fluoroalkyl amino acids) the synthesis of which is already known and has been appropriately studied.

The cyclopeptides were synthesised starting from a fluoromethylated amino acid in the form of a carboxylic ester; this is acylated with the bromide of the corresponding N-protected amino acid. After hydrolysis of the dipeptide ester thus obtained, the terminal carboxyl is condensed with H-Orn(Cbz)-Gly-OtBu.

After eliminating the protection of the nitrogen terminal from the tetrapeptide thus obtained, this is acylated with Fmoc-Allgly-OH (a precursor of aspartic acid) to give the totally protected linear pentapeptide. After deprotection of the two protective terminal groups, the process continues with cyclisation via TBTU, followed by oxidation of the allyl residue with permanganate. The last steps involve release of Cbz on the ornithine side chain, followed by guanidylation of the amine function, to obtain the final cyclopeptide, which is purified in RP-HPLC, with separation of the two diastereoisomers.

Described here below are some examples of preparations for the synthesis of fluorinated amino acids (building blocks):

### Preparation 1

H-(*R,*S)-α-Tfm-Phe-OEt
(*Burger, K., Gaa, K., Chemiker Zeitung, 1990, 114, 101-104*)

The product was prepared as described in the reference cited above.
¹H-NMR (CDCl₃) δ 7,33-7,18 (m, 5H, arom.), 4.26 (q, CH₂-CH₃), 3.45-2.95 (dd, CH₂-C₆H₅), 1.32 (t, CH₃).

### Preparation 2

H-(*R*,S)-α-Dfm-Phe-OEt
*(Bey, P., Vevert, J.P., Van Dorsselaer, V. and Kolb, M., J. Org. Chem. 1979, 44, 2732-42)*

The product was prepared as described in the reference cited above.
¹H-NMR (CDCl₃) δ 7.28-7.13 (m, 5H, arom.), 6.14-5.77 (t, CHF₂), 4.16 (q, CH₂-CH₃), 3.20-2.87 (2 dd, CH₂-C₆H₅), 1.21 (t, CH₃).

### Preparation 3

N-CH₃-(*R*,S)-α-Tfm-Phe-OCH₃
*(Buvger, K. and Hollweer, W., Synlett. 1994, 751-3)*

The product was prepared as described in the reference cited above.
¹H-NMR (CDCl₃) δ 7.28-7.15 (m, 5H, arom.), 3.73 (s, OCH₃), 3.27, 3.22, 3.16, 3.12 (q, CH₂-C₆H₅), 2.47 (s, N-CH₃).

These synthetic blocks (building blocks) are used for the synthesis of cyclopeptides according to the present invention, using techniques with which the technician with ordinary experience in the field will be familiar.

The following examples further illustrate the invention.

Abbreviations: TEA: triethylamine; THF: tetrahydrofuran; LDA: lithioisopropylamide; DMF: dimethylformamide; bromoe-namine: 1-bromo-N,N-2-trimethyl-1-propenylamine; HATU: O-(7-azabenzotriazol-1-yl)-N,N,N¹,N¹,N¹-tetramethyluronium hexafluorophosphate; DIEA: diisopropylamine; DCM: dicloromethane; DCC: dicyclohexylcarbodiimide; HOAT: azabenzotriazole; allgly: 2-allylglycine; Tfm: trifluoromethyl; Dfm: difluoromethyl; TBTU: O-(benzo-triazol-1-yl)N,N,N¹,N¹-tetramethyluronium tetrafluoroborate

### Example 1

### C(Arg-Gly-Asp-D-Phe-(R or S)-Tfm-Phe) (ST1930/ST1931)

### Preparation of Pht-D-Phe-Br (Dal Pozzo, A., Bergonzi, R. and Ni, M.H., Tetrahedron Lett., 2001, 42, 3925-7).

1.4 g (4.75 mmol) of Pht-D-Phe-OH are dissolved under argon in 19 ml of a 0.5 M solution of bromoenamine in DCM. After 10 minutes the solution is ready for use.

To 12.5 ml of the bromide solution (prepared as described above) cooled to 0°C, are added 248 mg (0.950 mmol) of H-α-Tfm-Phe-OEt and collidine (1 eq.); the mixture is stirred at room temperature (a.t.) and after 10 minutes another 6.5 ml of the bromide solution and 1 eq. of collidine are added. After 2 hours, the mixture is brought to dryness and extracted with 15 ml of NaHCO₃ 5% and 15 ml of EtOAc, leaving it to stir for 30 minutes. The solvent is washed with water, HCl 1N and water, followed by evaporation and purification of the residue on a flash chromatography column with hexane-EtOAc 8:2 as the solvent.

To a solution of 460 mg of the dipeptide Pht-D-Phe-α-Tfm-Phe-OEt in 21 ml of anhydrous DCM are added 4.3 ml of a 1M solution of BBr₃ in DCM; the mixture is countercurrent condensation heated for 2 hours and then washed with 21 ml of water and brought to dryness.

390 mg of the acid peptide (b) are dissolved in 6 ml of anhydrous CH₃CN with 406.7 mg (1.4 eq) of HATU and 2.8 eq of DIEA and left to stir for 15 minutes, after which 548 mg (2 eq) of HCl·H-Orn(Cbz)-OtBu and another 2 eq of DIEA are added. After 2 hours the reaction is diluted with 15 ml of DCM and extracted with 20 ml of brine. The organic phase is washed again with HCl 2N, NaHCO₃ and water. The residue is purified on a flash chromatography column with hexane-EtOAc 6:4 as the solvent.

424 mg of the tripeptide obtained above are dissolved in 2.2 ml of TFA/DCM (1:1) and brought to dryness after 30 minutes.

283.8 mg of the compound obtained are dissolved in 5.7 ml of DCM, and HCl·H-Gly-OtBu (1 eq), DIEA (2 eq), HOAT (3 eq) and DCC (3 eq) are added. After 20 minutes the mixture is filtered, the filtrate is washed with water, HCl 0.1N, NaHCO₃ 5% and water and then brought to dryness.

349 mg of the tetrapeptide obtained above are dissolved in 5 ml of EtOH, 600 µl of a 1M solution of NH₂-NH₂· H₂O in EtOH (1.5 eq) are added, and the reaction is countercurrent condensation heated for 2.5 hours. The EtOH is removed, and extraction is done with 10 ml of DCM and 10 ml of an aqueous solution containing 63.6 mg of Na₂CO₃ (1.5 eq). After 10 minutes' stirring, the organic phase is brought to dryness and the residue is purified by flash chromatography with CHCl₃-MeOH 98:2.

134 mg of the deprotected tetrapeptide are dissolved in 2.7 ml of DCM. Fmoc-AllGly-OH (1 eq), DIEA (1 eq), HOAT (1.2 eq) and DCC (1.2 eq) are added and the reaction then proceeds as described above.

151.6 mg of the pentapeptide obtained as described above are dissolved in 5 ml of DCM, and 140 µl of piperidine are added. After 2 hours the reaction mixture is washed with water, buffer pH 5.5, water, NaHCO₃ 5%, and water and then brought to dryness. The residue is purified by filtration on silica gel, washing first with CHCl₃ and then with CHCl₃-MeOH, 95:5.

The pentapeptide is deprotected at the carboxyl terminal as described above.

116 mg of the deprotected linear pentapeptide are dissolved in 86 ml of DMF, and TBTU (3 eq), HOBT (3 eq) and DIEA (860 µl) are added. After 5 minutes the mixture is brought to dryness, the residue is extracted with 10 ml of DCM and the solution washed with brine, HCl 2N, water, NaHCO₃ 5% and water.

80.9 mg of the cyclic pentapeptide, obtained as described above, are dissolved in 6.9 ml of acetone, the solution is cooled, and an aqueous solution of KMnO4 (100.5 mg in 620 µl) is added dropwise. After 1 hour at 0°C the reaction mixture is left overnight at room temperature. Finally, 770 µl of H₂SO₄ 3N and a solution of 40% NaHSO₃ are added until complete decoloration is achieved. After removing the acetone, the reaction is diluted with water and the product extracted with EtOAc.

66.8 mg of the cyclic pentapeptide are dissolved in 1 ml of a mixture of DMF/AcOH 6:4; 23.8 mg of ammonium formiate (5 eq) are added, and then 33.4 mg of Pd/C 10%. After 15 minutes the reaction mixture is diluted with MeOH and filtered on Celite and the filtrate is brought to dryness..

60.5 mg of the totally deprotected cyclic pentapeptide are dissolved in 540 µl of MeOH, 73 µl of DIEA (5 eq) and 49.8 mg of pyrazole-carboxamidine monochlorhydrate (4 eq) are added. After 1 hour the reaction is neutralised with TFA and brought to dryness. The purification and simultaneous separation of the 2 diastereoisomers were done by preparative RP-HPLC in the following conditions:
Column: Alltima (Alltech Italia) C18, 10 µm, 250 x 22 mm;
   Mobile phase: acetonitrile 34% in H₂O+0,1% TFA.
Flow rate: 12 ml/min.
   Diastereoisomer I (Rt 21.30) ST1930
   Diastereoisomer II (Rt 26.76) ST1931
Overall yield calculated on the basis of the last 3 steps: 33%.
Diastereoisomer I (ST1930)
   ¹H-NMR (CD₃OD): δ 7.30-7.19 (m, arom.), 4.70, 4.28 and 4.08 (m, α-CH), 3.80-3.15 (CH₂-Gly), 3.32 (m, CH₂-N), 3.15-2.80 (m CH₂-Asp + CH₂-Phe + CH₂-Tfm-Phe), 1.63-1.35 (m, CH₂-CH₂-Arg).
   ¹⁹F-NMR (CD₃OD): δ 5.18.
   MS (M+H⁺): 691.13.
Diastereoisimer II (ST1931)
   ¹H-NMR (CD₃OD): δ 7.23-7.20 (m, arom.), 4.85-4.05 (m, α-CH), 4.30-3.75 (2 dd CH₂-Gly), 3.18 (m, CH₂-N), 3.50-2.50 (m CH₂-Asp + CH₂-Phe + CH₂-Tfm-Phe), 2.10-1.55 (m, CH₂-CH₂-Arg).
   ¹⁹F-NMR (CD₃OD): δ 4.17.
   MS (M+H⁺): 691.13.

### Example 2

### c(Arg-Gly-Asp-D-Phe-(R,S)-Dfm-Phe) (ST1932)

Starting from H-(*R*,S)-Dfm-Phe-OEt, the process is as described in Example 1. A mixture of the two diastereoisomers, which have not been separated, is obtained.
¹H-NMR (CD₃OD): δ 8,00-7,05 (d, NH), 7.35-7.15 (m, arom.), 6.42-5.6 (t, CHF₂), 4.74-4.32 (m, α-CR), 4.32-3.96 (2 dd, CH₂-Gly), 3.68-2.50 (m), 1.75-1.45 (m, CH₂-CH₂-Arg).
¹⁹F-NMR (CD₃OD): δ from -54.4 to -55.5.
MS (M+H⁺): 673,14.

### Example 3

### c(Arg-Gly-(R or S)-Tfm-Asp-D-Phe-Val) (ST2189/2190)

H-(*R*,S)-Tfm-Allgly-Oet was condensed with Pth-Gly-Br, then proceeding as described in Example 1. The mixture of the two diastereoisomers was separated and purified by means of preparative HPLC with CH₃CN 22% in water + 0.1% TFA.
Diastereoisomer I (Rt 20.76)
   ¹H-NMR (DMSOD₆+D₂O): δ 7.30-7.12, 4.70, 4.26, 4.10 3.86, 3.60-2-70, 1.72, 1.52-1-12, 0.65.
   MS (M+H⁺): 643.2.
Diastereoisomer II (Rt 25.44)
   ¹H-NMR (DMSOD₆+D₂O): δ 7.30-6.96, 4.50, 4.15, 4.10-2.75, 1.90, 1.62, 1.43-1.20, 0.82.
   MS (M+H⁺): 643.2.

### Example 4

### c(Arg-Gly-Asp-(R o S)-Tfm-Phe-Val) (ST2191/2192)

HCl. H-(*R*,S)-Tfm-Phe-OEt was condensed with Pth-AllGly-Br, then proceeding as described in Example 1. The mixture of the two diastereoisomers was separated and purified by means of preparative HPLC with CH₃CN 30% in water + 0-1% TFA.
Diastereoisomer I (Rt 17.19)
   ¹H-NMR (D₂O): δ 7.50-7.30, 4.90, 4.30-4.16, 4.05 3.76-3.60, 3.32-2.82, 2.03-170, 1.57, 0.86, 0.73.
   MS (M+H⁺): 643.2.
Diastereoisomer II (Rt 22.17)
   ¹H-NMR (D₂O): δ 7.48-7.12, 4.75, 4.25, 4.07, 3.75-3.10, 2.95, 2.10, 1.85, 1.63-1.00, 0.77.
   MS (M+H⁺): 643.2.

### Example 5

### c(Arg-Gly-Asp-D-Phe-(R or S)-Tfm-VaD (ST2409/2410)

HCl·H-(*R,S*)-Tfm-Val-OEt was condensed with Pth-D-Phe-Br (being a hydrochloride a total of 3 eq of collidine were used). Then the process described in Example 1 was followed until obtaining of the tetrapeptide Pht-D-Phe-(R,S)-Tfm-Val-Orn(Cbz)-Gly-OtBu. The mixture of the two intermediate diastereoisomers was separated by means of flash chromatography [hexane-AcOEt = 4:6].
Diastereoisomer I (fast moving, Rf 0.23)
Diastereoisomer II (slow moving, Rf 0.36).

The two chirally pure intermediate products were treated separately with two parallel syntheses, with steps similar to those described in Example 1:
Diastereoisomer I (ST2409)
   ¹H-NMR (D₂O) δ 7.37-7.25; 4.83, 4.73, 4.46, 4.13, 3.47, 3.21, 3.08-2.69, 2.33, 1.92-1.50, 1.08.
   ¹⁹F-NMR (D₂O)δ 10.50, 0.97
   MS (M+H+): 643.16
Diastereoisomer II (ST2410)
   ¹H-NMR (D₂O) δ 7.37-7.29; 4.87, 4.73, 4.56, 4.46, 3.53, 3.18, 3.00-2.55, 1.87, 1.60, 1.18, 0.85.
   19F-NMR (D₂O) δ 9.29, 0.97
   MS (M+H+): 643.16

### Example 6

### c(Arg-Gly-Asp-D-Phe-(R or S)-N-Me-Tfm-Phe) [ST2552/2553]

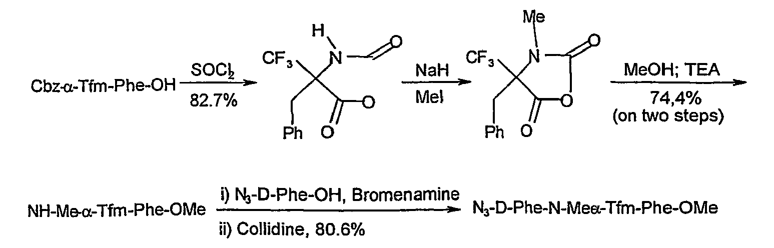

After hydrolysis of the ester at the terminal carboxyl, coupling with HOrn(Cbz)-Gly-OtBu was done; the azide group was then reduced with [Et₃NH][Sn(SPh)₃] and the linear pentapeptide was completed with condensation of Fmoc-Allgly at the N- terminal. As from this intermediate product on, the synthesis of the cyclopentapeptide continues as described in Example 1.

### Binding to integrin αᵥβ₃ receptors

96-well plates were subjected overnight to coating with 0.5 µg/ml of integrin αᵥβ₃ (Chemicon, cat. CC1020). On the next day, the wells were washed and incubation was performed with 0.05 nM of (¹²⁵I)Echistatin (Amersham, cat. IM304) in the absence or presence of compounds according to the invention. After a 3-hour incubation and a series of washings, the integrin from each well bound to the radioactive substance was solubilised with NaOH 2N, and the radioactivity was then measured using a gamma counter. Non-specific binding, to be subtracted from all samples, was determined in the presence of Echistatin 1 µM.

### Binding to integrin αᵥβ₅ receptors

96-well plates were subjected overnight to coating with 1 µg/ml of integrin αᵥβ₅ (Chemicon, cat. CC1022). On the next day, the wells were washed and incubation was performed with 0.05 nM of (¹²⁵I)Echistatin (Amersham, cat. IM304) in the absence or presence of compounds according to the invention. The process then proceeded as described in the previous case.

### Evaluation of IC₅₀ parameters

The affinity of the compounds according to the present invention for vitronectin receptors was expressed as the IC₅₀±SD value (nM) which is a parameter elaborated using "ALLFIT" software.

Table 1 here below shows the results obtained. In particular, ST2552 demonstrated a high binding affinity for both vitronectine receptors αᵥβ₃ and αᵥβ₅.

**Table 1**

| Compound | αᵥβ₃ | αᵥβ₅ |
|---|---|---|
| | IC50±SD (nM) | |
| ST1930 | 345±86 | 6.7±0.8 |
| ST1931 | 7238±1283 | 93±9 |
| ST1932 | 332±101 | 5.5±0.8 |
| ST2189 | >10,000 | >10,000 |
| ST2190 | >10,000 | >10,000 |
| ST2191 | 237±64 | 10±1.9 |
| ST2192 | 1018±348 | 72.7±3.8 |
| ST2409 | 36.3±0.84 | 3.4±0.07 |
| ST2410 | 285.3±8.1 | 42.8±0.5 |
| ST2552 | 18.9±0.7 | 7.7±0.1 |
| ST2553 | 704.4±9.2 | 93.3±1.9 |

### Cell cultures

Microvascular endothelial cells from bovine adrenal glands (bovine microvascular endothelial cells - BMEC) were isolated from animals that had just been sacrificed and were stored in ice up to their arrival in the laboratory. In sterile conditions, the glands were washed in a solution of betadine for 5 minutes and then in 2 liters of sterile PBS. The glands were then cut with a disposable sterile lancet into fragments of approximately 2 mm and transferred to polystyrene Falcon tubes containing PBS (30 ml per gland). After centrifuging at 600 rpm in a centrifuge cooled to +4°C, the supernatant was decanted. The pellet was resuspended 1:2 with a 0.12% solution of collagenase A (Boehringer Mannheim) and incubated at 37°C for 2 hours under stirring. After subsequent filtration on filters (Sigma), first of 200, and then 100 mesh, the supernatant was added to a solution of DMEM containing 15% FBS to inhibit the action of the collagenase A. The solution was centrifuged at 1000 rpm at room temperature and the precipitate resuspended in DMEM medium containing 20% FBS, 50 µg/ml of bovine brain extract (BBE), 50 µg/ml of heparin (Sigma), 0.5% v/v gentamicin (Sigma), and 1% v/v L-glutamine. The cells were seeded on Petri dishes gelatinised with 1% gelatin (Sigma porcine gelatin). At confluence, the cells were characterised with endothelial markers as Factor VIII.

Human melanoma MeWo cells from the American Type Culture Collection (ATCC) were kept in culture in complete Eagle Minimal Essential Medium (EMEM) containing 10% FCS, 2 mM L-glutamine and 50 µg/ml gentamicin.

SK-LMS-1 human leiomyosarcoma cells (ATCC) were grown in EMEM supplemented with 10% FCS, 2 mM L-glutamine and 50 µg/ml gentamicin.

All the cells were maintained at 37°C in a humidified atmosphere containing 5% CO₂.

### Cell adhesion assay

96-well plates were pretreated with 5 µg/ml of human vitronectin (Cal-biochem) for 12 hours at +4°C. BMEC or MeWo cells were detached with trypsin-EDTA, counted and plated on vitronectin substrate. The molecules were assayed at scalar concentrations ranging from 0.1 to 100 µM and co-incubated with the cells left to adhere. After incubation the cells were washed once with PBS with Ca²⁺ and Mg²⁺ so as to remove those that did not adhere to the substrate. The adhereing cells were fixed with a 4% paraformaldehyde solution for 10 minutes at room temperature. The cells were then stained with a 1% toluidine blue solution for 10 minutes at room temperature. After staining, the cells were washed in double-distilled water, dried and solubilised with a 1% SDS solution. The cells were then quantified by means of absorbance reading on a Victor multilabel plate counter (Wal-lac) at 600 nm.

The evaluation parameter for the activity of the molecules was the value IC₅₀±SD (µM).

Table 2 here below shows the results obtained with the molecules previously found to have a major binding affinity for vitronectine receptors. In particular, the molecule ST2552 showed to inhibit the MeWo human melanoma cells adesion to vitronectine with an IC50 value of 0.33 µM.

**Table 2**

| Compound | BMEC | MeWo | SK-LMS1 |
|---|---|---|---|
| | IC₅₀±SD (µM) | | |
| ST1930 | 8.9±0.4 | 6.9±1.3 | / |
| ST1932 | 14.2±1.0 | 13.9±4.3 | / |
| ST2191 | 18.8±4.4 | 21.9±2.5 | / |
| ST2192 | >100 | / | / |
| ST2409 | 12.5±1.5 | / | 15.9±1.6 |
| ST2410 | 77.6±7.2 | / | >100 |
| ST2552 | >100 | 0.33±0.05 | / |

In keeping with another object of the present invention, the pharmaceutical compositions contain at least one formula (I) compound as an active ingredient, in an amount such as to produce a significant therapeutic effect. The compositions covered by the present invention are entirely conventional and are obtained with methods which are common practice in the pharmaceutical industry, such as, for example, those illustrated in *Remington's Pharmaceutical Science Handbook, Mack Pub. N.Y.* - latest edition. According to the administration route chosen, the compositions will be in solid or liquid form, suitable for oral, parenteral or intravenous administration. The compositions according to the present invention contain, along with the active ingredient, at least one pharmaceutically acceptable vehicle or excipient. These may be particularly useful formulation coadjuvants, e.g. solubilising agents, dispersing agents, suspension agents, and emulsifying agents.

## Claims

1. Compounds of formula (I)
cyclo[NX₁-R₁-CO-NX₂-R₂-CO-NX₃-R₃-CO-NX₄-R₄-CO-NX₅-R₅-CO]
where:
R₁ is selected from:
CH(CH₂)₃NHC(NH)NH₂; C[CHₙFₘ](CH₂)₃NHC(NH)NH₂
R₂ is the group CH₂; CH₂-CH₂; 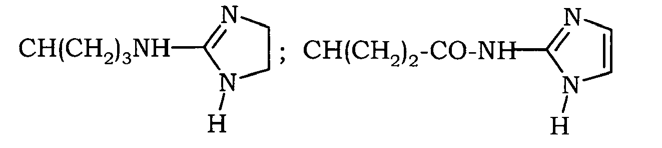
R₃ is selected from CHCH₂COOH; C[CHₙFₘ]CH₂-COOH;
R₄ is selected from CH-CH₂-Ph; C[CHₙFₘ]CH₂-Ph; CH-CH₂-(4-OH)Ph; CH-CH₂-(4-OMe)Ph; CH-CH₂-(4-F)Ph; CH-CH(OH)-Ph; C(CH₃)₂; CH-C(CH3)₃; CH-CH₂-COOH; 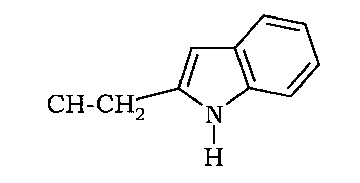
R₅ is selected from CH-CH₂-Ph; C[CHₙFₘ]CH₂-Ph; CH-CH(CH₃)₂; C[CHₙFₘ]CH(CH₃)₂; CH-C(CH₃)₃;
or, the group NX₄-R₄-CO-NX₅-R₅-CO is 3-aminomethyl-benzoyl n+m=3
X₁-X₅, which may be the same or different, are H, (CH₂)ₙ-CH₃; 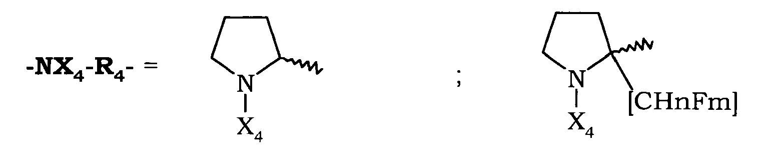 (CH₂)ₙ-CHF₂; (CH₂)ₙ-CH₂F, (CH₂)ₙ-CF₃ where n = 0-3;
with the proviso that there is at least one α-fluoroalkylated amino acid present in the formula (I) compound;
where each NX-R-CO amino acid can have an absolute type R or type S configuration; their individual enantiomers, diastereoisomers, the related mixtures, the pharmaceutically acceptable salts.

2. Compound according to claim 1, selected from the group consisting of:
c(Arg-Gly-Asp-D-Phe-(*R* or S)-Tfm-Phe);
c(Arg-Gly-Asp-D-Phe-(*R*,S)-Dfm-Phe);
c(Arg-Gly-Asp-(*R or* S)-Tfm-Phe-Asp-D-Phe-Val);
c(Arg-Gly-Asp-(*R or S*)-Tfm-Phe-Val);
c(Arg-Gly-Asp-D-Phe-(*R* or S)-Tfm-Val)
c(Arg-Gly-Asp-D-Phe-(*R* or *S*)-N-Me-Tfm-Phe.

3. Compounds according to claims 1 or 2 for use as medicaments.

4. Use of the compounds according to claims 1 or 2 for the preparation of medicaments that inhibit the receptors belonging to the family of the integrins belonging to the αᵥβ₃ and αᵥβ₅ system.

5. Use according to claim 4, where said medicaments have antiangiogenic activity.

6. Use according to claim 5, where said medicaments have antimetastatic activity.

7. Use according to claim 5, where said medicaments are useful for the treatment of a disease selected from the group consisting of retinopathy, acute kidney failure, and osteoporosis.

8. Pharmaceutical compositions containing at least one compound according to claims 1 or 2 as an active ingredient in a mixture with pharmaceutically acceptable vehicles and/or excipients.

9. Use of compounds according to claims 1-2 for the preparation of dia gnostic agents for detecting and locating small tumour masses or for the analysis of arterial occlusion events such as strokes or myocardial infarcts, wherein said compounds are labelled.

10. Use according to claim 9, where said diagnostic agent is used for the detection and location of tumour masses.

11. Use according to claim 10, where said tumour masses are small.

12. Use according to claim 9, where said diagnostic agent is used for detecting and locating arterial occlusion events.

13. Use according to claim 12, where said event is a stroke or myocardial infarct.

14. A diagnostic agent containing at least one compound according to claims 1 or 2.

## Patentansprüche

1. Verbindungen der Formel (I)
cyclo[NX₁-R₁-CO-NX₂-R₂-CO-NX₃-R₃-CO-NX₄-R₄-CO-NX₅-R₅-CO]
worin
R₁ ausgewählt ist aus:
CH(CH₂)₃NHC(NH)NH₂; C[CHₙFₘ](CH₂)₃NHC(NH)NH₂
R₂ die Gruppe CH₂; CH₂-CH₂; 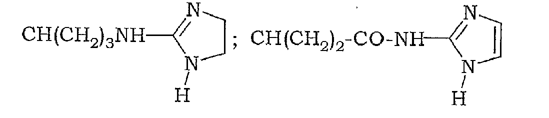 ist;
R₃ ausgewählt ist aus CHCH₂COOH; C[CHₙFₘ]CH₂-COOH;
R₄ ausgewählt ist aus CH-CH₂-Ph; C[CHₙFₘ]CH₂-Ph; CH-CH₂-(4-OH)Ph; CH-CH₂-(4-OMe)Ph; CH-CH₂-(4-F)Ph; CH-CH(OH)-Ph; C(CH₃)₂; CH-C(CH3)₃; CH-CH₂-COOH; 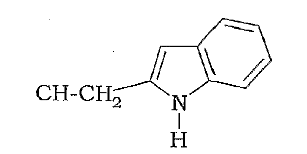
R₅ ausgewählt ist aus CH-CH₂-Ph; C[CHₙFₘ]CH₂-Ph; CH-CH(CH₃)₂; C[CHₙFₘ]CH(CH₃)₂; CH-C(CH₃)₃;
oder die Gruppe NX₄-R₄-CO-NX₅-R₅-CO 3-Aminomethyl-benzoyl ist n + m = 3
X₁-X₅, welche gleich oder verschieden sein können, H, (CH₂)ₙ-CH₃ sind; 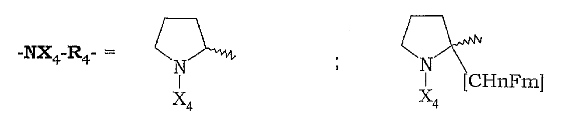 (CH₂)ₙ-CHF₂; (CH₂)ₙ-CH₂F, (CH₂)ₙ-CF₃, worin n = 0-3 ist;
mit der Maßgabe, dass wenigstens eine α-fluoralkylierte Aminosäure in der Verbindung der Formel (I) vorhanden ist;
worin jede NX-R-CO Aminosäure die absolute Konfiguration vom S- oder R-Typ haben kann; deren jeweilige Enantiomere, Diastereomere, die zugehörigen Mischungen, die pharmazeutisch annehmbaren Salze.

2. Verbindungen gemäß Anspruch 1, ausgewählt aus der Gruppe bestehend aus:
c(Arg-Gly-Asp-D-Phe-(*R* or S)-Tfm-Phe);
c(Arg-Gly-Asp-D-Phe-(*R*,S)-Dfm-Phe);
c(Arg-Gly-Asp-(*R or* S)-Tfm-Phe-Asp-D-Phe-Val);
c(Arg-Gly-Asp-(*R or* S)-Tfm-Phe-Val);
c(Arg-Gly-Asp-D-Phe-(*R or* S)-Tfm-Val)
c(Arg-Gly-Asp-D-Phe-(*R or S*)-N-Me-Tfm-Phe.

3. Verbindungen gemäß den Ansprüchen 1 oder 2 zur Verwendung als Arzneimittel.

4. Verwendung der Verbindungen gemäß den Ansprüchen 1 oder 2 für die Herstellung von Arzneimitteln, welche die Rezeptoren inhibieren, die zur Familie der Integrine gehören, die zu dem αᵥβ₃- und αᵥβ₅-System gehören.

5. Verwendung gemäß Anspruch 4, wobei die Arzneimittel antiangiogenische Aktivität haben.

6. Verwendung gemäß Anspruch 5, wobei die Arzneimittel antimetastatische Aktivität haben.

7. Verwendung gemäß Anspruch 5, wobei die Arzneimittel für die Behandlung von Erkrankungen nützlich sind, die ausgewählt sind aus der Gruppe bestehend aus Retinopathie, akutem Nierenversagen und Osteoporose.

8. Pharmazeutische Zusammensetzungen enthaltend wenigstens eine Verbindung gemäß den Ansprüchen 1 oder 2 as wirksamen Bestandteil in einer Mischung mit pharmazeutisch annehmbaren Trägern und/oder Hilfsstoffen.

9. Verwendung von Verbindungen gemäß den Ansprüchen 1-2 für die Herstellung von diagnostischen Agenzien zum Nachweis und zur Lokalisierung kleiner Tumormassen oder zur Untersuchung von arteriellen Verschlusserkrankungsereignissen, wie Schlaganfall oder Myokardinfarkten, worin die Verbindungen markiert sind.

10. Verwendung gemäß Anspruch 9, wobei das diagnostische Agens zum Nachweis und zur Lokalisierung von Tumormassen verwendet wird.

11. Verwendung gemäß Anspruch 10, wobei die Tumormassen klein sind.

12. Verwendung gemäß Anspruch 9, wobei das diagnostische Agens zum Nachweis und zur Lokalisierung von arteriellen Verschlusserkrankungsereignissen verwendet wird.

13. Verwendung gemäß Anspruch 12, wobei das Ereignis ein Schlaganfall oder Myokardinfarkt ist.

14. Diagnostisches Agens enthaltend wenigstens eine Verbindung gemäß den Ansprüchen 1 oder 2.

## Revendications

1. Composés de formule (I)
cyclo[NX₁-R₁-CO-NX₂-R₂-CO-NX₃-R₃-CO-NX₄-R₄-CO-NX₅-R₅-CO]
dans laquelle :
R₁ est choisi parmi :
CH(CH₂)₃NHC(NH)NH₂; C[CHₙFₘ](CH₂)₈NHC(NH)NH₂
R₂ est le groupe CH₂ ; CH₂-CH₂ ; 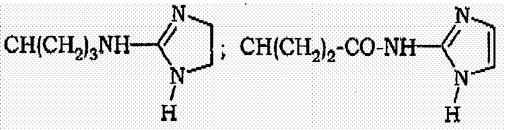
R₃ est choisi parmi CHCH₂COOH; C[CHₙFₘ]CH₂-COOH;
R₄ est choisi parmi CH-CH₂-Ph; C[CHₙFₘ]CH₂-Ph; CH-CH₂-(4-OH)Ph; CH-CH₂-(4-OMe)Ph; CH-CH₂-(4-F)Ph; CH-CH(OH)-Ph; C(CH₃)₂; CH-C(CH₃)₃; CH-CH₂-COOH; 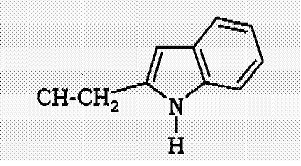
R₅ est choisi parmi CH-CH₂-Ph; C[CHₙFₘ]CH₂-Ph; CH-CH(CH₃)₂; C[CHₙFₘ]CH(CH₃)₂; CH-C(CH₃)₃;
ou, le groupe NX₄-R₄-CO-NX₅-R₅-CO est 3-aminométhylbenzoyle
n + m = 3
X₁ à X₅, qui peuvent être identiques ou différents, sont H, (CH₂)ₙ-CH₃; 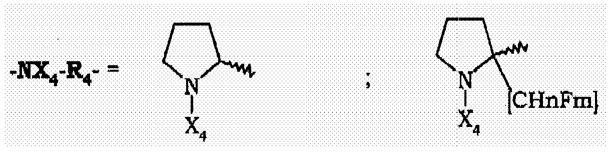 (CH₂)ₙ-CHF₂; (CH₂)ₙ-CH₂F, (CH₂)ₙ-CF₃ où n = 0 à 3 ;
avec pour condition qu'il y ait au moins un acide aminé α-fluoroalkylé présent dans le composé de formule (I) ;
où chaque acide aminé NX-R-CO peut avoir une configuration absolue de type R ou de type S ; leurs énantiomères individuels, les diastéréoisomères, les mélanges apparentés, les sels pharmaceutiquement acceptables.

2. Composé selon la revendication 1, choisi dans le groupe constitué par :
c(Arg-Gly-Asp-D-Phe-(*R* ou S)-Tfm-Phe);
c(Arg-Gly-Asp-D-Phe-(*R,*S)-Dfm-Phe);
c(Arg-Gly-Asp-(*R* ou S)-Tfm-Phe-Asp-D-Phe-Val);
c(Arg-Gly-Asp-(*R* ou S)-Tfm-Phe-Val);
c(Arg-Gly-Asp-D-Phe-(*R* ou S)-Tfm-Val)
c(Arg-Gly-Asp-D-Phe-(*R* ou *S*)-N-Me-Tfm-Phe.

3. Composés selon les revendications 1 ou 2, pour l'utilisation en tant que médicaments.

4. Utilisation des composés selon les revendications 1 ou 2, pour la préparation de médicaments qui inhibent les récepteurs appartenant à la famille des intégrines appartenant au système αᵥβ₃ et αᵥβ₅.

5. Utilisation selon la revendication 4, dans laquelle lesdits médicaments ont une activité antiangiogénique.

6. Utilisation selon la revendication 5, dans laquelle lesdits médicaments ont une activité antimétastatique.

7. Utilisation selon la revendication 5, dans laquelle lesdits médicaments sont utiles pour le traitement d'une maladie choisie dans le groupe constitué par une rétinopathie, une insuffisance rénale aiguë et l'ostéoporose.

8. Compositions pharmaceutiques contenant au moins un composé selon les revendications 1 ou 2 en tant qu'ingrédient actif dans un mélange avec des véhicules et/ou excipients pharmaceutiquement acceptables.

9. Utilisation de composés selon les revendications 1-2 pour la préparation d'agents diagnostiques pour détecter et localiser de petites masses tumorales ou pour l'analyse d'événements d'occlusion artérielle tels que des attaques ou des infarctus du myocarde, dans laquelle lesdits composés sont marqués.

10. Utilisation selon la revendication 9, dans laquelle ledit agent diagnostique est utilisé pour la détection et la localisation de masses tumorales.

11. Utilisation selon la revendication 10, dans laquelle lesdites masses tumorales sont petites.

12. Utilisation selon la revendication 9, dans laquelle ledit agent diagnostique est utilisé pour détecter et localiser des événements d'occlusion artérielle.

13. Utilisation selon la revendication 12, dans laquelle ledit événement est une attaque ou un infarctus du myocarde.

14. Agent diagnostique contenant au moins un composé selon les revendications 1 ou 2.
